# EUROPÄISCHE PATENTSCHRIFT

(11) **EP 4 518 809 B1**
(45) Veröffentlichungstag und Bekanntmachung des Hinweises auf die Patenterteilung: **22.04.2026**
(21) Anmeldenummer: 23725617.7
(22) Anmeldetag: 05.05.2023
(51) Int. Cl.: A61F 2/76

(54) **VERFAHREN ZUM EINRICHTEN EINER ORTHOPÄDIETECHNISCHEN EINRICHTUNG**
METHOD FOR SETTING UP AN ORTHOPAEDIC APPLIANCE
PROCÉDURE D'INSTALLATION D'UN APPAREILLAGE ORTHOPÉDIQUE

(30) Priorität: 06.05.2022 DE 102022111327
(43) Veröffentlichungstag der Anmeldung: 12.03.2025
(73) Patentinhaber: Ottobock SE & Co. KGaA, 37115 Duderstadt (DE)
(72) Erfinder: DAUR, Christian, 37115 Duderstadt (DE); LEINIGER, Andreas, 37115 Duderstadt (DE); KÄPPEL, Christian, 37115 Duderstadt (DE); MOSLER, Lüder, 37115 Duderstadt (DE)
(74) Vertreter: Gramm, Lins & Partner Patent- und Rechtsanwälte PartGmbB
(86) Internationale Anmeldenummer: PCT/EP2023/061964
(87) Internationale Veröffentlichungsnummer: WO 2023/214026

(56) Entgegenhaltungen:
- KR-A- 20200 013 157
- US-A1- 2017 360 578
- US-A1- 2018 243 155
- US-A1- 2021 069 984
- GIORGIO COLOMBO ET AL: "A digital patient for computer-aided prosthesis design", INTERFACE FOCUS, vol. 3, no. 2, 6 April 2013 (2013-04-06), GB, pages 20120082, XP055671164, ISSN: 2042-8898, DOI: 10.1098/rsfs.2012.0082

## Beschreibung

Die Erfindung betrifft ein Verfahren zum computer-basierten Einrichten einer orthopädietechnischen Einrichtung, die am Körper eines damit ausgerüsteten Patienten getragen wird. Die Erfindung betrifft ebenso ein Computerprogramm hierzu.

Orthopädietechnische Einrichtungen sind im Sinne der vorliegenden Erfindung insbesondere Orthesen, Prothesen, Exoskelette und ggf. auch Rollstühle sowie die zugehörigen Sitzschalen und Sitzkissen, die individuell an den Nutzer angepasst werden. Orthesen sind Produkte, die ein Körperteil des Patienten, beispielsweise ein Gelenk, stützen, unterstützen, schützen oder in der Bewegungsfreiheit einschränken, um eine Überbeanspruchung zu vermeiden. Prothesen hingegen ersetzen nicht oder nicht mehr vorhandene Körperteile des Patienten. Exoskelette sind insbesondere mechanische Stützstrukturen, die den Hauptbewegungsapparat des Patienten stützen, unterstützen oder schützen sollen.

Unter einem Patienten wird im Folgenden jeder Verwender der orthopädietechnischen Einrichtung verstanden. Es handelt sich folglich um den Träger der orthopädietechnischen Einrichtung.

In der Regel wird eine orthopädietechnische Einrichtung an einem Körperteil des Patienten angeordnet. Dabei muss es nicht zwangsläufig mit der Haut des Patienten in Kontakt kommen. Orthesen und Exoskelette werden beispielsweise häufig über der Kleidung getragen, sodass diese, beispielsweise eine Hose, sich zwischen der orthopädietechnischen Einrichtung und der Haut des Patienten befindet. Dennoch wird beispielsweise eine Knie-Orthese am Knie oder am Bein des Patienten befestigt. Eine Prothese verfügt immer über ein mit einem Amputationsstumpf oder einem anderen Körperteil verbundenes Schnittstellenelement, das an dem jeweiligen Körperteil befestigt wird. Bei einer Beinprothese wird beispielsweise ein Prothesenschaft verwendet, der die Schnittstelle zwischen der Prothese und dem Amputationsstumpf darstellt. In diesem Fall wäre der Amputationsstumpf das Körperteil des Patienten. Zwischen der Hautoberfläche des Amputationsstumpfes des Patienten und dem Prothesenschaft wird in der Regel ein Liner verwendet, um auf die Haut wirkende Scherkräfte zu reduzieren.

Ein Prothesenschaft für einen Amputationsstumpf wird in der Regel aus einem starren (kaum verformbaren) Material, beispielsweise einem faserverstärkten Kunststoff, hergestellt und bildet einen wichtigen Teil des Interfaces zwischen dem Amputationsstumpf und der Prothese, die am Prothesenschaft angeordnet wird. Insbesondere für Beinprothesen, die an einem Amputationsstumpf, beispielsweise einem Oberschenkelstumpf, angeordnet werden sollen, werden entsprechende Prothesenschäfte seit langem verwendet.

Insbesondere Prothesenschäfte für Beinamputierte sind im täglichen Gebrauch besonderen Belastungen ausgesetzt. Beim Gehen lastet das gesamte Gewicht des Patienten auf dem Prothesenschaft und damit insbesondere auf dem Amputationsstumpf, der in dem Prothesenschaft angeordnet wird. Es ist daher von größter Wichtigkeit, den Prothesenschaft möglichst optimal an die individuellen Gegebenheiten und Bedürfnisse des Patienten anzupassen, insbesondere an die Form und Geometrie des betreffenden Körperteils, wobei insbesondere die unterschiedlichen Bewegungssituationen wie Gehen, Stehen oder Hinsetzen zu berücksichtigen sind.

Sowohl beim Aufbau also bei der Einrichtung einer orthopädietechnischen Einrichtung wird der Patient von einer meist technisch geschulten Fachkraft unterstützt, beispielsweise einem Orthopädietechniker. Mit seinem Wissen und seiner Erfahrung kann der Orthopädietechniker die notwendigen Einstellungen vornehmen, damit die orthopädietechnische Einrichtung optimal passt und ein schonendes Bewegungsbild ergibt.

Aus der EP 2 153 370 B1 ist ein System zum Ausrichten von Prothesen bekannt, bei dem sowohl Bewegungsdaten der mit der Prothese ausgerüsteten Person als auch Prothesenausrichtungsfehler aus einer Bewegungsdatenbank ermittelt werden. Diese werden miteinander verglichen um festzustellen, ob die Prothese einem Soll-Wert entspricht oder weiter eingestellt werden muss.

Aus der DE 10 2012 009 507 A1 ist ein Verfahren und eine Vorrichtung zur Bestimmung von Fehlstellungen im Aufbau von Prothesen der unteren Extremitäten bekannt, wobei Inertialmessdaten über zumindest einen Gangzyklus ermittelt und mit Sollwerten verglichen werden.

Aus der DE 10 2018 128 514 B4 ist ein Verfahren zum Durchführen eines statischen Prothesenaufbaus einer Prothese bekannt, wobei mehrere Komponenten aneinander angeordnet werden. Dabei werden eine Ist-Position und eine Ist-Orientierung der aneinander angeordneten Komponenten relativ zueinander anhand erfasster Positionen und Orientierungen von Markierungen ermittelt und mit entsprechenden Sollwerten verglichen. Hierbei werden lediglich die Bauteile untereinander verglichen, ohne die individuelle Situation des Patienten zu berücksichtigen.

Beim Einrichten einer orthopädietechnischen Einrichtung, dass den Aufbau und die Auswahl einer orthopädietechnischen Einrichtung, das Anpassen einer vorhandenen orthopädietechnischen Einrichtung sowie das Einstellen von Parametern der orthopädietechnischen Einrichtung umfasst, muss sichergestellt werden, dass die orthopädietechnische Einrichtung so eingerichtet ist, dass keine negativen Auswirkungen auf die übrigen Körperteile oder den gesamten Körper entstehen. Das Einrichten kann weiterhin auch die automatische Anpassung und/oder Erstellung von individualisierten Herstellungsdaten für die orthopädietechnische Einrichtung, beispielsweise für eine anschließende additive Fertigung, beinhalten.

Aus der US 2021/069984 A1 wird das Erstellen einer orthopädietechnischen Einrichtung offenbart. Dabei werden zunächst Patientendaten bereitgestellt, aus denen dann ein Patientenmodell ermittelt bzw. generiert wird. Aus diesem Patientenmodell werden dann Patientenparameter abgeleitet, wobei basierend darauf dann eine virtuelle Repräsentation der orthopädietechnischen Einrichtung unter Anwendung von Geräteparametern erstellt werden soll. Es können nun Patientenparameter oder Geräteparameter angepasst werden, um die Auswirkung der Änderung in der visuellen Repräsentation zu erkennen. Diese Änderungen werden dabei von einem Benutzer, beispielsweise einem Orthopädietechniker, getätigt, wobei basierend auf dem fertigen Modell dann die entsprechende orthopädietechnische Einrichtung hergestellt wird.

Aus GIORGIO COLOMBO ET AL: "A digital patient for computer-aided prosthesis design", INTERFACE FOCUS, Bd. 3, Nr. 2, 6. April 2013 (2013-04-06), Seite 20120082, x9055671164, GB ISSN: 2042-8898, DOI: 10.1098/rsfs.2012.0082 ist das computergestützte Erstellung einer Prothese bekannt. Hierfür wird zunächst basierend auf Patientendaten ein digitaler Patient erstellt, der zur Visualisierung durch einen Avatar repräsentiert wird. Mithilfe von entsprechenden Modellen wird dabei auch der zu versorgende Stumpf erstellt. Mithilfe eines Modell-Frameworks wird dann ein entsprechendes Prothesenmodell unter Berücksichtigung des modellierten Stumpfes erstellt. Dabei wird unter anderem auch der Prothesenschaft automatisch erstellt.

Die KR20200013157A offenbart das Herstellen einer individuellen und angepassten Knöchel- oder Fußorthese. Dabei wird zunächst die Form und Größe des Schenkels des Patienten vermessen. Anschließend wird eine an diesen Schenkel angepasste Orthese modelliert, wobei dann Eingang des Patienten simuliert wird. Basierend auf dieser Gangsimulation werden dann Druckstellen identifiziert, an denen dann zusätzliche Polsterung und vorgesehen werden. Anschließend kann die Orthese in einem 3D-Drucker hergestellt werden.

Die US 2018/0243155 A1 offenbart das Erstellen eines Exoskeletts. Dabei wird zunächst ein Körperscan des Patienten durchgeführt, der das Exoskelett tragen soll. Anschließend wird basierend auf dem Körperscan ein vollständiges 3D-Oberflächenmodell des Patienten erstellt. Dieses Oberflächenmodell wird nun verwendet, um das Exoskelett hieran anzupassen bzw. ein entsprechendes Exoskelett angepasst an dieses Oberflächenmodell zu erstellen.

Die US2017/360578 A1 offenbart schließlich ebenfalls das Erstellen einer Prothese oder einer Orthese, wobei auch hier zunächst ein Körpermodell erstellt wird.

Vor diesem Hintergrund ist es Aufgabe der vorliegenden Erfindung ein verbessertes Verfahren zum Einrichten einer orthopädietechnischen Vorrichtung vorzuschlagen.

Die Aufgabe wird mit dem Verfahren zum Einrichten einer orthopädietechnischen Einrichtung gemäß Anspruch 1 erfindungsgemäß gelöst. Vorteilhafte Ausgestaltungen der Erfindung finden sich in den entsprechenden Unteransprüchen.

Gemäß Anspruch 1 wird ein Verfahren zum computer-basierten Einrichten einer orthopädietechnischen Einrichtung, die am Körper eines damit ausgerüsteten Patienten getragen wird, vorgeschlagen, wobei das Verfahren die folgenden, durch eine Datenverarbeitungsanlage ausgeführten oder ausführbaren Schritte umfasst:
- Bereitstellen eines digitalen Körpermodells;
- Bereitstellen von körperbezogenen Patientenparametern für die Versorgung des Patienten mit einer orthopädietechnischen Einrichtung;
- Bereitstellen eines digitalen orthopädietechnischen Versorgungsvorschlags, der die Versorgung des Patienten mit einem orthopädietechnischen Versorgungsmodell und hierauf bezogenen Versorgungsparametern umfasst;
- Simulieren einer orthopädietechnischen Versorgung des Patienten an dem Körpermodell mit dem orthopädietechnischen Versorgungsvorschlag unter Berücksichtigung der bereitgestellten körperbezogenen Patientenparameter mittels einer Simulationseinrichtung,
- wobei basierend auf der Simulation eine Wechselwirkung zwischen dem Körpermodell und dem orthopädietechnischen Versorgungsvorschlag mittels einer Auswerteeinheit ermittelt wird; und
- Bestimmen eines optimierten orthopädietechnischen Versorgungsvorschlags basierend auf dem bereits bereitgestellten orthopädietechnischen Versorgungsvorschlags in Abhängigkeit von der ermittelten Wechselwirkung mittels der Auswerteeinheit.

Demnach wird erfindungsgemäß vorgeschlagen, dass eine orthopädietechnische Versorgung des Patienten an dem bereitgestellten digitalen Körpermodell mit dem bereitgestellten orthopädietechnischen Versorgungsvorschlag simuliert wird, wobei bei der Simulation des orthopädietechnischen Versorgungsvorschlags die bereitgestellten körperbezogenen Patientenparameter berücksichtigt werden. Die Simulation ermittelt dabei eine Wechselwirkung zwischen dem Körpermodell und dem Versorgungsvorschlag und kann so die Auswirkungen des bereitgestellten Versorgungsvorschlags auf das digitale Körpermodell ermitteln. Dadurch kann bereits vor der Versorgung des Patienten mit dem Versorgungsvorschlag festgestellt werden, welche Auswirkungen der Versorgungsvorschlag mit den vorgeschlagenen Einstellparametern auf den Patienten haben könnte bzw. haben wird.

Basierend auf der ermittelten Wechselwirkung zwischen dem digitalen Körpermodell und dem digitalen Versorgungsvorschlag wird nun durch die Auswerteeinheit der Datenverarbeitungsanlage ein optimierter orthopädietechnischer Versorgungsvorschlag ermittelt. Der optimierte orthopädietechnische Versorgungsvorschlag wird dabei bspw. durch Anpassen/Verändern des bereitgestellten ursprünglichen Versorgungsvorschlags ermittelt. Der optimierte orthopädietechnische Versorgungsvorschlag beinhaltet dabei insbesondere Maßnahmen zum Einrichten der physischen orthopädietechnischen Einrichtung dahingehend, dass das Versorgungsziel bestmöglich erreicht wird und zusätzlich negative Auswirkungen auf den Körper des Patienten verringert, reduziert oder gänzlich vermieden werden.

Wurde ein optimierter orthopädietechnischer Versorgungsvorschlag durch die Auswerteeinheit bestimmt, so kann dieser auf einem Display der Datenverarbeitungsanlage angezeigt werden. Dabei kann die Fachkraft gegebenenfalls einen weiteren Simulationsdurchlauf starten, bis ein aus der Sicht der Fachkraft maximaloptimierter orthopädietechnischer Versorgungsvorschlag erreicht wurde.

Basierend auf einem durch das Verfahren ermittelten optimierten orthopädietechnischen Versorgungsvorschlag wird nun die orthopädietechnische Einrichtung des Patienten eingerichtet, damit die so eingerichtete orthopädietechnische Einrichtung am Körper des Patienten getragen werden kann. Die orthopädietechnische Einrichtung des Patienten wird demnach so eingerichtet, wie es der optimierte orthopädietechnische Versorgungsvorschlag aus der Simulation vorschlägt.

Damit wird es erstmals möglich, negative Auswirkungen auf den Körper des Patienten beim Einrichten einer orthopädietechnischen Einrichtung ohne den Patienten zu ermitteln, sodass dem Patienten eine optimal eingerichtete orthopädietechnische Einrichtung angepasst werden kann.

Das bereitgestellte digitale Körpermodell kann dabei ein generisches Körpermodell sein, welches durch die körperbezogenen Patientenparameter individualisiert wird. Das Körpermodell kann dabei insbesondere ein digitales (standardisiertes) anatomisches Körpermodell sein.

Gemäß einer Ausführungsform ist vorgesehen, dass der optimierte orthopädietechnische Versorgungsvorschlag als Grundlage für eine erneute Simulation der orthopädietechnischen Versorgung verwendet wird.

Der durch die Simulation aufgefundene optimierte Versorgungsvorschlag wird somit als Grundlage für eine erneute Simulation verwendet, indem die in dem aufgefundenen optimierten Versorgungsvorschlag enthaltenen Maßnahmen zum Einrichten der orthopädietechnischen Einrichtung auf den zuvor bereitgestellten Versorgungsvorschlag, welcher der Simulation zugrunde gelegt wurde, angewendet werden. Hierdurch kann die Simulation iterativ sich einem Optimum (global oder lokal) annähern und ein bestmögliches Ergebnis liefern.

Gemäß einer Ausführungsform ist vorgesehen, dass der bereitgestellte orthopädietechnische Versorgungsvorschlag zuvor automatisch in Abhängigkeit von zumindest einem Teil der körperbezogenen Patientenparameter mittels einer Recheneinheit erstellt wird.

Anhand der Patientenparameter, welche auch pathologische Informationen enthalten können, kann die Recheneinheit automatisch einen orthopädietechnischen Versorgungsvorschlag erstellen, bspw. anhand der körperbezogenen Informationen des Patienten, die der Recheneinheit zuvor bereitgestellt wurden. So kann beispielsweise unter Kenntnis entsprechender Amputationsinformationen ein für eine entsprechende Prothese Versorgungsvorschlag erstellt werden, die an die körperbezogenen Patientenparameter und den darin enthaltenen Informationen angepasst ist.

Dabei kann in einer Datenbank oder einem maschinellen Lernsystem für verschiedene Kombinationen von Patientenparameter entsprechende voreingestellt Versorgungsvorschläge hinterlegt sein. Basierend auf konkreten Patientenparameter wird nun aus dieser Vielzahl von voreingestellten Versorgungsvorschlägen einer ausgewählt und der Simulation zugrunde gelegt. Dabei kann ebenfalls vorgesehen sein, dass durch die Recheneinheit der voreingestellte Versorgungsvorschlag vor der Simulation basierend auf den Patientenparametern entsprechend angepasst wird.

Gemäß einer Ausführungsform ist vorgesehen, dass das digitale Körpermodell zumindest einen Teil des menschlichen Skeletsystems, bedarfsweise auch mindestens eine Gelenkfunktion hierzu, modelliert.

Dabei kann vorgesehen sein, dass zur Simulation der Gelenkfunktion auch eine Simulation der Muskeln, Sehnen und Bänder durchgeführt wird und gegebenenfalls auch deren Ansteuerung durch die Nerven, was insbesondere aufgrund von medizinischen Einschränkungen relevant sein kann.

Gemäß einer Ausführungsform ist vorgesehen, dass das digitale Körpermodell in Abhängigkeit von zumindest einem Teil der körperbezogenen Patientenparameter an den Patienten angepasst ist oder wird und/oder dass das digitale Körpermodell anhand von Messdaten einer zuvor durchgeführten Vermessung des physischen Patientenkörpers individualisiert ist oder wird.

Dabei kann vorgesehen sein, dass das digitale Körpermodell als generisches Körpermodell bereitgestellt wird und durch die körperbezogenen Patientenparameter an die Besonderheiten des Patienten angepasst wird. Das digitale Körpermodell kann aber auch individuell anhand von Messdaten einer zuvor durchgeführten Vermessung individualisiert werden, wodurch ein noch besseres Abbild des Patienten der Simulation zugrundegelegt werden kann. Die Vermessung kann dabei beispielsweise aus dem Abnehmen einzelner Maße am Patienten oder aber auch einem Teilweise oder vollständigen 3D-Scan bestehen. Bevorzugt werden möglichst viele Informationen auch abseits des zu versorgenden Bereichs zur Verfügung gestellt, bspw. Informationen über Körperteile, die den Versorgungsvorschlag nicht unmittelbar betreffen (bspw. angrenzende Gelenke).

Gemäß einer Ausführungsform ist vorgesehen, dass das Bestimmen eines optimierten orthopädietechnischen Versorgungsvorschlags das Verändern der Versorgungsparameter des orthopädietechnischen Versorgungsmodells und/oder die Auswahl des orthopädietechnischen Versorgungsmodells umfasst.

Der Versorgungsvorschlag kann demnach Maßnahmen enthalten, welche den der Simulation zugrunde gelegten Versorgungsvorschlag derart verändern, dass insbesondere negative Wechselwirkungen auf bestimmte Körperteile oder den gesamten Körper des Patienten verringert, reduziert oder gänzlich vermieden werden, während gleichzeitig das Versorgungsziel bestmöglich erreicht wird. Derartige Maßnahmen können beispielsweise das Einstellen von bestimmten Parametern darstellen, mit der die orthopädietechnische Einrichtung eingestellt werden kann. Dies betrifft bei (mikroprozessorgesteuerten) Prothesen beispielsweise bestimmte Dämpfungseigenschaften, Winkelstellungen oder ähnlichen Maßnahmen. Je komplexer die Prothese dabei ist, desto mehr Parameter lassen sich üblicherweise modifizieren, was insbesondere bei mikroprozessorgesteuerten Prothesen auch automatisiert von der Software durchführbar ist.

Der optimierte Versorgungsvorschlag kann indes jedoch auch Maßnahmen enthalten, welche die Auswahl eines bestimmten orthopädietechnischen Versorgungsmodells umfassen. So kann es geboten sein, basierend auf den simulierten Wechselwirkungen und der sich daraus ergebende Auswirkung auf den Körper des Patienten das Versorgungsvorschlag zugrunde liegende orthopädietechnische Versorgungsmodell zu ändern und gegen ein anderes Versorgungsmodell auszutauschen, beispielsweise ein Versorgungsmodell eines anderen Typs.

Gemäß einer Ausführungsform ist vorgesehen, dass bei der Simulation ein statischer Lastfall mit der simulierten orthopädietechnischen Versorgung simuliert wird, wobei als Wechselwirkung zwischen dem Körpermodell und dem orthopädietechnischen Versorgungsvorschlag Druckstellen, Anlagepunkte, Anlageflächen und/oder Abweichungen des Gesamtaufbaus von einem vorgegebenen Aufbau der orthopädietechnischen Versorgung ermittelt werden.

Bei einem statischen Lastfall wird eine Beanspruchung der orthopädietechnischen Einrichtung simuliert, bei der die orthopädietechnische Einrichtung nicht bewegt wird und stattdessen durch eine statische Kraft beansprucht wird. Dies ist insbesondere beim Stehen, Sitzen und Liegen der Fall. Durch das Simulieren des statischen Lastfalls können Wechselwirkungen zwischen dem Körpermodell und dem Versorgungsvorschlag statischer Art ermittelt werden, wie beispielsweise Druckstellen und Anlagepunkten, die dem Patienten Schmerzen bereiten könnten. Hierauf basierend kann dann ein optimierter Versorgungsvorschlag erstellt werden, der beispielsweise Maßnahmen vorsieht, um diese Druckstellen und Anlagepunkte abzumildern und so den Komfort für den Patienten zu erhöhen oder auch bestehende Gelenkfehlstellungen zu beheben. Dies ist insoweit auch deshalb erforderlich, da hierdurch Schonhaltungen vermieden werden.

Gemäß einer Ausführungsform ist vorgesehen, dass bei der Simulation ein dynamischer Lastfall mit der simulierten orthopädietechnischen Versorgung simuliert wird, bei dem mittels der orthopädietechnischen Versorgung eine bestimmte Bewegung oder ein bestimmter Bewegungsablauf simuliert wird, wobei als Wechselwirkung zwischen dem Körpermodell und dem orthopädietechnischen Versorgungsvorschlag eine Belastung der betroffenen Gelenke, ein Bewegungsumfang und/oder ein Abweichung zwischen der simulierten Bewegung und einer vorgegebenen optimalen Bewegung oder zwischen dem simulierten Bewegungsablauf und einem vorgegebenen optimalen Bewegungsablauf ermittelt werden.

Bei dem dynamischen Lastfall wird eine Bewegung oder ein Bewegungsablauf simuliert und dabei die Wechselwirkung bei der Bewegung zwischen dem Körpermodell und dem Versorgungsvorschlag ermittelt. Dies betrifft insbesondere die Belastung der betroffenen Gelenke, den Bewegungsumfang sowie Abweichungen von einem optimalen Bewegungsablauf. Basierend auf der so festgestellten Wechselwirkung und den negativen Auswirkungen auf den Körper des Patienten kann dann ein entsprechender optimierter Versorgungsvorschlag mit Maßnahmen erstellt werden, der diese negativen Auswirkungen abschwächt bzw. verhindert.

Erfindungsgemäß ist vorgesehen, dass als Wechselwirkung zwischen dem Körpermodell und dem orthopädietechnischen Versorgungsvorschlag eine Auswirkung eines statischen oder dynamischen Lastfalls mit der simulierten orthopädietechnischen Versorgung auf wenigstens ein Gelenk ermittelt wird, welches von dem unmittelbar versorgten Gelenk verschieden ist und/oder welches innerhalb einer lastfallbezogenen Gelenkkette des Körpermodells vorgesehen ist.

Es wird somit bei der Wechselwirkung zwischen dem Körpermodell und dem Versorgungsvorschlag nicht nur auf das unmittelbar versorgte Gelenk abgestellt, sondern es werden auch jene Gelenke berücksichtigt, die davon verschieden sind bzw. die innerhalb einer lastfallbezogenen Gelenkkette vorgesehen sind. So kann es beispielsweise notwendig sein, bei der Simulation einer Knieorthese auch die Auswirkungen auf das Hüftgelenk oder die Wirbelsäule zu betrachten, wenn ein optimierter Versorgungsvorschlag erstellt werden soll. Gelenke einer Gelenkkette sind dabei jene Gelenke, die ausgehend von dem versorgten Gelenk bzw. dem ersetzten Gelenk maßgeblich an dem Bewegungsablauf beteiligt und notwendig sind.

Neben den lastbezogenen Wechselwirkungen zwischen orthopädietechnischer Versorgung und Körpermodell können auch weitere Wechselwirkungen einbezogen werden, beispielsweise die Ansteuerbarkeit der Versorgung durch die verbleibende Muskelkraft. Insbesondere bei EMG gesteuerten Prothesen oder Orthesen kann die Wechselwirkung zwischen Muskelsignalen des Modells und der Sensorik der Versorgung betrachtet werden, um so die Sensorik optimal auszurichten.

Gemäß einer Ausführungsform ist vorgesehen, dass der optimierte orthopädietechnische Versorgungsvorschlag mindestens eine Maßnahme an einem anderen als dem direkt versorgten Körperteil und/oder Gelenk enthält.

Der optimierte orthopädietechnische Versorgungsvorschlag kann demnach auch Maßnahmen enthalten, die sich auf andere Körperteile oder Gelenke beziehen, um die sich aus der simulierten Wechselwirkung zwischen dem Körpermodell und dem Versorgungsvorschlag ergebenden negativen Auswirkungen auf den Körper des Patienten abzuschwächen oder zu verhindern.

In einer weiteren Ausführungsform ist es denkbar, dass ein Vergleich zwischen einem generischen Körpermodell ohne Parametrisierung durch die Patientenparameter und einem generischen Körpermodell mit Parametrisierung durch die Patientenparameter erfolgt. Dabei wird eine Simulation mit dem bereitgestellten orthopädietechnischen Versorgungsvorschlag in Bezug auf das generische Körpermodell ohne Parametrisierung und eine Simulation mit dem bereitgestellten orthopädietechnischen Versorgungsvorschlag in Bezug auf das generische Körpermodell mit Parametrisierung durchgeführt, wobei beide Simulationen verglichen werden und anhand des Vergleiches dann die Wechselwirkung ermittelt wird, um so einen optimierten orthopädietechnischen Versorgungsvorschlag zu bestimmen.

Die Erfindung wird anhand der beigefügten Figuren beispielhaften erläutert. Es zeigen:
- Figur 1: schematische Darstellung des Verfahrens in Bezug zur ausführenden Datenverarbeitungsanlage;
- Figur 2: schematische Darstellung eines generischen Körpermodells in Form eines Avatars;
- Figur 3: schematische Darstellung einer statischen Simulation in einer ersten Ausführungsform;
- Figur 4: schematische Darstellung einer statischen Simulation in einer zweiten Ausführungsform;
- Figur 5: schematische Darstellung einer dynamischen Simulation.

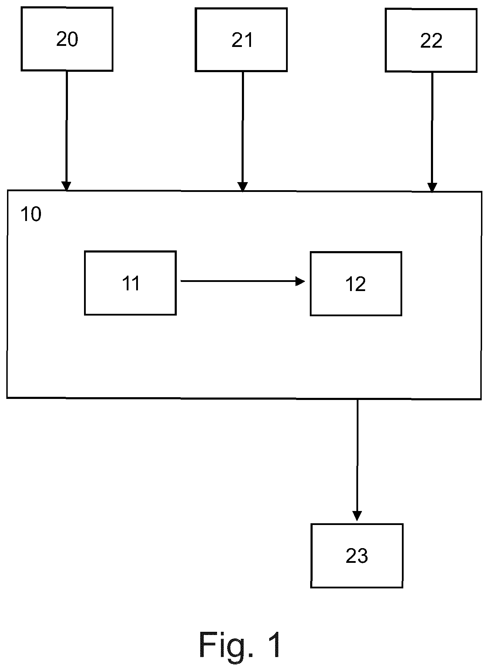

Figur 1 zeigt in einer schematisch stark vereinfachten Darstellung eine Datenverarbeitungsanlage 10, die eine Simulationseinrichtung 11 und eine Auswerteeinheit 12 hat. Die Simulationseinrichtung 11 und die Auswerteeinheit 12 können dabei Softwaremodule darstellen, die auf der Datenverarbeitungsanlage 10 ausgeführt werden und dabei in Wechselwirkung miteinander stehen, um das vorstehend beschriebene Verfahren ausführen zu können.

Der Datenverarbeitungsanlage 10 wird nun zunächst ein digitales Körpermodell 20 bereitgestellt, welches beispielsweise ein generisches Körpermodell sein kann. Des Weiteren werden der Datenverarbeitungsanlage 10 entsprechende körperbezogene Patientenparameter 21 bereitgestellt, welches sich auf entsprechende körperbezogene Besonderheiten des Patienten beziehen. Das generische digitale Körpermodell 20 kann dabei mithilfe der bereitgestellten körperbezogenen Patientenparameter 21 dahingehend ergänzt werden, dass das Körpermodell i.V.m. den Patientenparametern den Körper des Patienten modelliert.

Schließlich wird der Datenverarbeitungsanlage 10 ein digitaler orthopädietechnischer Versorgungsvorschlag 22 bereitgestellt, der im Zusammenhang mit dem Körpermodell und den Patientenparametern simuliert werden soll.

Das Körpermodell 20, die Patientenparameter 21 sowie der Versorgungsvorschlag 22 können dabei der Datenverarbeitungsanlage 10 auch über eine Datenbank bereitgestellt werden, in der die einzelnen Daten abgespeichert sind.

Mithilfe der Simulationseinrichtung 11 wird nun der bereitgestellte orthopädietechnische Versorgungsvorschlag 22 an dem Körpermodell 20 mit den parametrisierten Patientenparameter 21 simuliert, wobei hierbei sowohl ein statischer als auch ein dynamischer Lastfall simuliert werden kann. Basierend auf dieser Simulation wird durch die Auswerteeinheit 12 dann eine Wechselwirkung zwischen dem Körpermodell und dem Versorgungsvorschlag ermittelt, um daraus dann einen optimierten orthopädietechnischen Versorgungsvorschlag 23 bestimmen zu können.

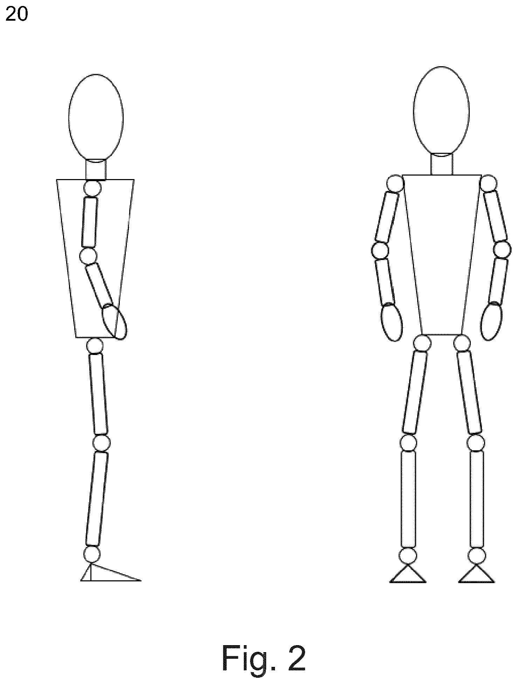

Figur 2 zeigt in einer schematischen Darstellung ein digitales Körpermodell 20, dass auf der linken Seite in einer Seitenansicht und auf der rechten Seite in einer Frontalansicht dargestellt ist. Das digitale Körpermodell 20 ist in Figur 2 in Form eines generischen Körpermodells dargestellt und weist insbesondere die für eine Bewegung notwendigen Gelenke und Körperteile auf. Das digitale Körpermodell 20 ist dabei so implementiert, dass es für jedes Gelenk entsprechende Bewegungseinschränkungen hat, umso die Bewegung eines Menschen zu modellieren.

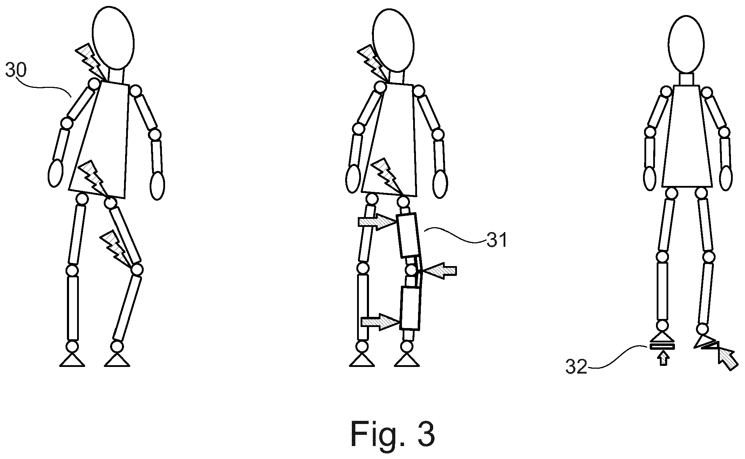

Figur 3 zeigt ein Ausführungsbeispiel, wie ein orthopädietechnischer Versorgungsvorschlag basierend auf der Simulation ermittelt wird. Auf der linken Seite ist dabei das Körpermodell 30 gezeigt, welches durch die Patientenparameter entsprechend parametrisiert ist.

Der Patient leidet dabei sowohl an einem linken O-Bein als auch an einer Beinlängendifferenz. Diese Patientendaten werden dabei in Form von Patientenparameter dem generischen Körpermodell 20 zugeführt, um daraus dass der Figur 3 ersichtliche patientenbezogene Körpermodell 30 zu erstellen (linke Darstellung). Zu erkennen ist hierbei, dass sowohl in dem linken Knie, in dem hier linken Hüftgelenk als auch im Halswirbelbereich bei einer Simulation entsprechende Beschwerden auftreten.

In der mittleren Ansicht ist das Körpermodell 30 gezeigt, dass mit einem Versorgungsvorschlag 31 zur Behandlung des O-Beins ausgerüstet ist. Diese Kombination aus patientenbezogenem Körpermodell 30 und dem damit ausgerüsteten Versorgungsvorschlag 31 wird nun mithilfe der statischen Simulation simuliert, um die Wechselwirkung dieses Versorgungsvorschlages 31 auf den übrigen Körper des Patienten zu ermitteln.

Dies ist in der mittleren Ansicht dadurch gekennzeichnet, dass sowohl im linken Hüftgelenk als auch im Halswirbelbereich für den Patienten auch mit dem vorgeschlagenen Versorgungsvorschlag 31 weiterhin Beschwerden bestehen dürften.

Durch die Simulation und die damit einhergehende Auswertung der Simulation wird dann durch die Auswerteeinheit festgestellt, dass durch den Versorgungsvorschlag 31 zwar die Beschwerden im linken Knie behoben wurden, nicht jedoch die aus der Beinlängendifferenz bestehenden Beschwerden im linken Hüftgelenk und im Halswirbelbereich.

Der daraus entstehende optimierte orthopädietechnische Versorgungsvorschlag sieht daher Maßnahmen vor, die Beinlängendifferenz auszugleichen. Der optimierte Versorgungsvorschlag sieht daher vor, Einlagen 32 zu verwenden, um die Beschwerden des Patienten abzumildern.

Der optimierte orthopädietechnische Versorgungsvorschlag sieht daher vor, die Knie-orthese 31 durch Einlagen 32 zu ersetzen, um sowohl die O-Bein bedingte Fehlstellung des linken Knies als auch die Beschwerden im linken Hüftgelenk und im Halswirbelbereich zu adressieren.

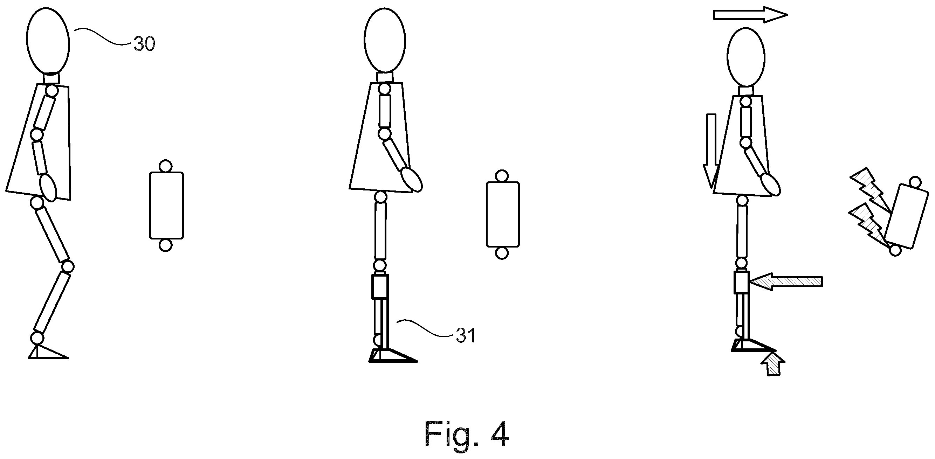

Figur 4 zeigt ein Ausführungsbeispiel, bei dem auf der linken Seite das Körpermodell 30 wieder mit den Patientenparameter parametrisiert wurde. In der mittleren Ansicht wurde der Patient mit einem Versorgungsvorschlag 31 am Körpermodell simuliert, wobei der Patient einmal in der Seitenansicht und einmal in der Draufansicht zu sehen ist. Die Simulation zeigt, dass zwar die statischen Lastfälle ohne Befund sind, jedoch in dynamischen Lastfall eine Kompensationsbewegung der Hüfte und der Wirbelsäule stattfindet. Im hier dargestellten Fall führt die Nachbildung der Zehenabstoßung durch die Orthese zu einer Überstreckung des Knies. Die Hüfte wird auf der betroffenen Seite als Kompensationsbewegung nach hinten gedreht. Dies führt weiterhin zu einer ungewollten Rotation in der Wirbelsäule und möglichen langfristigen Schädigungen an den Zwischenwirbelkörpern. Dies ist bei einer lokalen Betrachtung des betroffenen Bereiches kaum bis gar nicht zu erkennen und würde somit zu einer für den Patienten ungünstigen Versorgung führen.

Der optimierte orthopädietechnische Versorgungsvorschlag kann dann vorsehen, den Versorgungsvorschlag 31 entsprechend so einzustellen, dass die bestehende Kompensationsbewegung der Hüfte und der Wirbelsäule nicht mehr stattfindet. Hierzu können entsprechende Parameter an der simulierten orthopädietechnischen Einrichtung eingestellt werden, die dann die Beschwerden abmildert. Im vorliegenden Beispiel könnte eine geringfügige Beugung des Unterschenkels eingestellt werden, die die Korrekturwirkung der Orthese reduziert und die Überkorrektur vermeidet. Alternativ könnte eine Absatzerhöhung der Orthese erfolgen. Eine weitere Alternative wäre die Verwendung einer weicheren oder kürzeren Fußplatte, wodurch der vordere Hebelarm verkürzt und die Dynamik positiv beeinflusst würde. Das System kann die verschiedenen Alternativen vergleichen und auch in weiteren Simulationsrunden prüfen. So ließe sich beispielsweise feststellen, dass die Absatzhöhenanpassung zwar den statischen Fall weiter verbessert, jedoch keine Lösung für die Probleme im dynamischen Fall liefert und daher zu verwerfen ist.

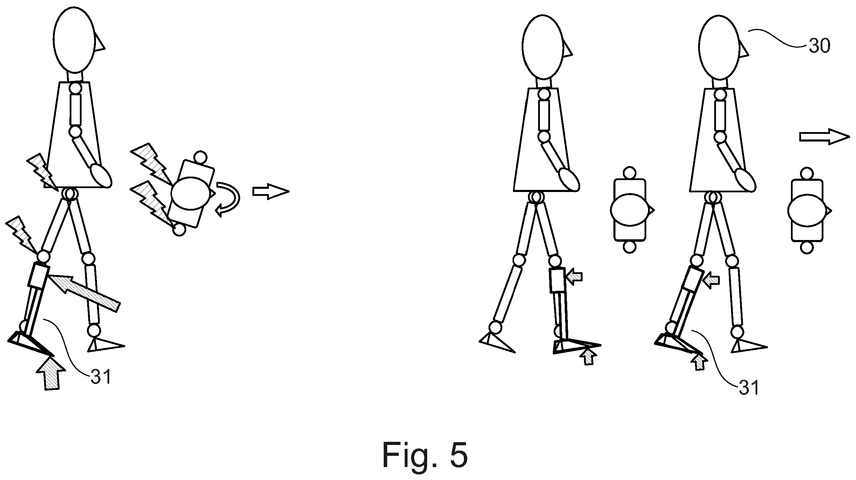

Figur 5 zeigt ein Ausführungsbeispiel, bei dem ein dynamischer Lastfall an einem parametrisierten Körpermodell 30 mit einem Versorgungsvorschlag 31 simuliert wird. In der linken Ansicht ist zu erkennen, dass der bereitgestellte Versorgungsvorschlag 31 mit seinen eingestellten Parametern überkorrigiert, wodurch Beschwerden im Knie sowie im Hüftgelenk und der Wirbelsäule entstehen.

Auf der rechten Seite ist ein optimierter Versorgungsvorschlag 31 zu erkennen, der keine negativen Wechselwirkungen mehr im dynamischen Lastfall zeigt. Der optimierte orthopädietechnische Versorgungsvorschlag kann dabei Maßnahmen enthalten, die zu einer Korrektur der eingestellten Parameter des Versorgungsvorschlages 31 raten, um das Überkorrigieren abzumildern.

### Bezugszeichenliste

10 Datenverarbeitungsanlage
11 Simulationseinrichtung
12 Auswerteeinheit
20 digitales Körpermodell
21 Patientenparameter
22 bereitgestellter digitaler orthopädietechnischer Versorgungsvorschlag
23 optimierte orthopädietechnische Versorgungsvorschlag
30 parametrisiertes Körpermodell
31 digitale Versorgungsvorschlag

## Patentansprüche

1. Verfahren zum computer-basierten Einrichten einer orthopädietechnischen Einrichtung, die am Körper eines damit ausgerüsteten Patienten getragen wird, wobei das Verfahren die folgenden computerimplementierten Schritten umfasst:
- Bereitstellen eines digitalen Körpermodells (20);
- Bereitstellen von körperbezogenen Patientenparametern (21) für die Versorgung des Patienten mit einer orthopädietechnischen Einrichtung;
- Bereitstellen eines digitalen orthopädietechnischen Versorgungsvorschlags (22), der die Versorgung des Patienten mit einem orthopädietechnischen Versorgungsmodell und hierauf bezogenen Versorgungsparametern umfasst;
- Simulieren einer orthopädietechnischen Versorgung des Patienten an dem Körpermodell (20) mit dem orthopädietechnischen Versorgungsvorschlag (22) unter Berücksichtigung der bereitgestellten körperbezogenen Patientenparameter (21) mittels einer Simulationseinrichtung (11),
- wobei basierend auf der Simulation eine Wechselwirkung zwischen dem Körpermodell (20) und dem orthopädietechnischen Versorgungsvorschlag (22) mittels einer Auswerteeinheit (12) ermittelt wird, und
- Bestimmen eines optimierten orthopädietechnischen Versorgungsvorschlags (23) basierend auf dem bereits bereitgestellten orthopädietechnischen Versorgungsvorschlags (22) in Abhängigkeit von der ermittelten Wechselwirkung mittels der Auswerteeinheit (12), **dadurch gekennzeichnet dass** als Wechselwirkung zwischen dem Körpermodell und dem orthopädietechnischen Versorgungsvorschlag (22) eine Auswirkung eines statischen oder dynamischen Lastfalls mit der simulierten orthopädietechnischen Versorgung auf wenigstens ein Gelenk ermittelt wird, welches von einem unmittelbar versorgten Gelenk verschieden ist und/oder welches innerhalb einer lastfallbezogenen Gelenkkette des Körpermodells (20) vorgesehen ist.

2. Verfahren nach Anspruch 1, **dadurch gekennzeichnet, dass** der optimierte orthopädietechnische Versorgungsvorschlag (23) als Grundlage für eine erneute Simulation der orthopädietechnischen Versorgung verwendet wird.

3. Verfahren nach Anspruch 1 oder 2, **dadurch gekennzeichnet, dass** der bereitgestellte orthopädietechnische Versorgungsvorschlag (22) zuvor automatisch in Abhängigkeit von zumindest einem Teil der körperbezogenen Patientenparameter (21) mittels einer Recheneinheit erstellt wird.

4. Verfahren nach einem der vorhergehenden Ansprüche, **dadurch gekennzeichnet, dass** das digitale Körpermodell (20) zumindest einen Teil des menschlichen Skeletsystems, bedarfsweise auch mindestens eine Gelenkfunktion hierzu, modelliert.

5. Verfahren nach einem der vorhergehenden Ansprüche, **dadurch gekennzeichnet, dass** das digitale Körpermodell (20) in Abhängigkeit von zumindest einem Teil der körperbezogenen Patientenparameter (21) an den Patienten angepasst ist oder wird und/oder dass das digitale Körpermodell (20) anhand von Messdaten einer zuvor durchgeführten Vermessung des physischen Patientenkörpers individualisiert ist oder wird.

6. Verfahren nach einem der vorhergehenden Ansprüche, **dadurch gekennzeichnet, dass** das Bestimmen eines optimierten orthopädietechnischen Versorgungsvorschlags (23) das Verändern der Versorgungsparameter des orthopädietechnischen Versorgungsmodells und/oder die Auswahl des orthopädietechnischen Versorgungsmodells umfasst.

7. Verfahren nach einem der vorhergehenden Ansprüche, **dadurch gekennzeichnet, dass** bei der Simulation ein statischer Lastfall mit der simulierten orthopädietechnischen Versorgung simuliert wird, wobei als Wechselwirkung zwischen dem Körpermodell und dem orthopädietechnischen Versorgungsvorschlag (22) Druckstellen, Anlagepunkte, Anlageflächen und/oder Abweichungen des Gesamtaufbaus von einem vorgegebenen Aufbau der orthopädietechnischen Versorgung ermittelt werden.

8. Verfahren nach einem der vorhergehenden Ansprüche, **dadurch gekennzeichnet, dass** bei der Simulation ein dynamischer Lastfall mit der simulierten orthopädietechnischen Versorgung simuliert wird, bei dem mittels der orthopädietechnischen Versorgung eine bestimmte Bewegung oder ein bestimmter Bewegungsablauf simuliert wird, wobei als Wechselwirkung zwischen dem Körpermodell (20) und dem orthopädietechnischen Versorgungsvorschlag (22) eine Belastung der betroffenen Gelenke, ein Bewegungsumfang und/oder ein Abweichung zwischen der simulierten Bewegung und einer vorgegebenen optimalen Bewegung oder zwischen dem simulierten Bewegungsablauf und einem vorgegebenen optimalen Bewegungsablauf ermittelt werden.

9. Verfahren nach einem der vorhergehenden Ansprüche, **dadurch gekennzeichnet, dass** der optimierte orthopädietechnische Versorgungsvorschlag (23) mindestens eine Maßnahme an einem anderen als dem direkt versorgten Körperteil und/oder Gelenk enthält.

10. Computerprogramm mit Programmcodemitteln eingerichtet zur Durchführung des Verfahrens nach einem der vorhergehenden Ansprüche, wenn das Computerprogramm auf einer Datenverarbeitungsanlage (10) ausgeführt wird.

## Claims

1. A method for computer-based setup of an orthopedic device wearing on the body of a patient equipped with it, the method comprising the following computer-implemented steps:
- providing a digital body model (20);
- providing body-related patient parameters (21) for the care of the patient with an orthopedic device;
- providing a digital orthopedic care proposal (22) comprising an orthopedic care model and care parameters related thereto for the care of the patient;
- simulating orthopedic care for the patient on the body model (20) with the orthopedic care proposal (22), taking into account the provided patient-related body parameters (21), using a simulation device (11),
- wherein, based on the simulation, an interaction between the body model (20) and the orthopedic care proposal (22) is determined using an evaluation unit (12), and
- determining an optimized orthopedic care proposal (23) based on the orthopedic care proposal (22) already provided, depending on the interaction determined by means of the evaluation unit (12),
**characterized in that** the interaction between the body model and the orthopedic care proposal (22) is determined as the effect of a static or dynamic load case with the simulated orthopedic care on at least one joint which is different from a directly cared joint and/or which is provided within a load case-related joint chain of the body model (20).

2. Method according to claim 1, **characterized in that** the optimized orthopedic care proposal (23) is used as the basis for a new simulation of the orthopedic care.

3. Method according to claim 1 or 2, **characterized in that** the provided orthopedic care proposal (22) is automatically generated in advance as a function of at least one part of the body-related patient parameters (21) by means of a computing unit.

4. Method according to one of the preceding claims, **characterized in that** the digital body model (20) models at least a part of the human skeletal system, and, if necessary, at least one joint function thereof.

5. Method according to one of the preceding claims, **characterized in that** the digital body model (20) is or becomes adapted to the patient depending on at least a part of the body-related patient parameters (21) and/or that the digital body model (20) is or becomes individualized on the basis of measurement data from a previously performed measurement of the physical patient body.

6. Method according to one of the preceding claims, **characterized in that** determining an optimized orthopedic care proposal (23) comprises changing the care parameters of the orthopedic care model and/or selecting the orthopedic care model.

7. Method according to one of the preceding claims, **characterized in that** during the simulation, a static load case is simulated with the simulated orthopedic care, whereby the interaction between the body model and the orthopedic care proposal (22) is determined as pressure points, contact points, contact surfaces, and/or deviations of the overall structure from a predetermined structure of the orthopedic care.

8. Method according to one of the preceding claims, **characterized in that** during the simulation, a dynamic load case is simulated with the simulated orthopedic care, in which a specific movement or a specific sequence of movements is simulated with the orthopedic care, whereby the interaction between the body model (20) and the orthopedic care proposal (22) is determined as a load on the affected joints, a range of motion, and/or a deviation between the simulated movement and a specified optimal movement or between the simulated movement sequence and a specified optimal movement sequence.

9. Method according to one of the preceding claims, **characterized in that** the optimized orthopedic care proposal (23) contains at least one measure on a body part and/or joint other than the one directly cared.

10. Computer program with program code means set up to carry out the method according to one of the preceding claims when the computer program is executed on a data processing system (10).

## Revendications

1. Procédé pour l'installation assistée par ordinateur d'un dispositif orthopédique porté sur le corps d'un patient qui en est équipé, le procédé comprenant les étapes suivantes mises en œuvre par ordinateur et consistant à :
- fournir un modèle de corps numérique (20) ;
- fournir des paramètres (21) liés au corps du patient, nécessaires pour équiper le patient d'un dispositif orthopédique ;
- fournir une proposition d'équipement orthopédique numérique (22) qui comprend l'équipement du patient d'un modèle d'équipement orthopédique et les paramètres d'équipement liés à celui-ci ;
- simuler l'équipement orthopédique du patient sur le modèle de corps (20) à l'aide de la proposition d'équipement orthopédique (22), en tenant compte des paramètres fournis (21) liés au corps du patient, au moyen d'un dispositif de simulation (11) ;
- déterminer, à partir de la simulation, l'interaction entre le modèle de corps (20) et la proposition d'équipement orthopédique (22) au moyen d'une unité d'évaluation (12), et
- définir une proposition d'équipement orthopédique optimisée (23) à partir de la proposition d'équipement orthopédique (22) initialement fournie, en fonction de l'interaction déterminée, au moyen de l'unité d'évaluation (12),
**caractérisé en ce que**
l'on détermine, comme interaction entre le modèle de corps et la proposition d'équipement orthopédique (22), l'effet d'un cas de charge statique ou dynamique appliquée par l'équipement orthopédique simulé sur au moins une articulation qui est différente de l'articulation directement équipée et/ou qui fait partie d'une chaîne articulaire du modèle de corps (20) soumise au cas de charge.

2. Procédé selon la revendication 1,
**caractérisé en ce que** la proposition d'équipement orthopédique optimisée (23) sert de base à une nouvelle simulation de l'équipement orthopédique.

3. Procédé selon la revendication 1 ou 2,
**caractérisé en ce que** la proposition d'équipement orthopédique (22) fournie est générée automatiquement au préalable au moyen d'une unité de calcul à partir d'une partie au moins des paramètres (21) liés au corps du patient.

4. Procédé selon l'une des revendications précédentes,
**caractérisé en ce que** le modèle de corps numérique (20) modélise au moins une partie du système squelettique humain et, selon les besoins, également au moins une fonction articulaire de celui-ci.

5. Procédé selon l'une des revendications précédentes,
**caractérisé en ce que** le modèle de corps numérique (20) est adapté au patient en fonction d'au moins une partie des paramètres (21) liés au corps du patient, et/ou **en ce que** le modèle de corps numérique (20) est individualisé ou on procède à une telle individualisation à partir de données de mesure issues d'une mesure préalablement effectuée du corps physique du patient.

6. Procédé selon l'une des revendications précédentes,
**caractérisé en ce que** la définition d'une proposition d'équipement orthopédique optimisée (23) comprend la modification des paramètres d'équipement du modèle d'équipement orthopédique et/ou la sélection du modèle d'équipement orthopédique.

7. Procédé selon l'une des revendications précédentes,
**caractérisé en ce que** la simulation implique un cas de charge statique avec l'équipement orthopédique simulé, et on détermine, comme interaction entre le modèle de corps et la proposition d'équipement orthopédique (22), les points de pression, les points de contact, les surfaces de contact et/ou les écarts de la structure globale par rapport à une structure donnée de l'équipement orthopédique.

8. Procédé selon l'une des revendications précédentes,
**caractérisé en ce que** la simulation implique un cas de charge dynamique avec l'équipement orthopédique simulé, où un mouvement spécifique ou une séquence de mouvements spécifique est simulé(e) au moyen de l'équipement orthopédique, et on détermine, comme interaction entre le modèle de corps (20) et la proposition d'équipement orthopédique (22), une charge sur les articulations concernées, une amplitude de mouvement et/ou un écart entre le mouvement simulé et un mouvement optimal donné ou entre la séquence de mouvements simulée et une séquence de mouvements optimale donnée.

9. Procédé selon l'une des revendications précédentes,
**caractérisé en ce que** la proposition d'équipement orthopédique optimisée (23) comprend au moins une action à effectuer sur une partie du corps et/ou une articulation autre que celle directement équipée.

10. Programme informatique doté de moyens de code de programme, conçu pour mettre en œuvre le procédé selon l'une des revendications précédentes, lorsque le programme informatique est exécuté sur un système de traitement de données (10).
